(19) Europäisches Patentamt
European Patent Office
Office européen des brevets

(11) Veröffentlichungsnummer: **0 247 537 B1**

# (12) EUROPÄISCHE PATENTSCHRIFT

(45) Veröffentlichungstag der Patentschrift: **04.09.91**

(51) Int. Cl.⁵: **C07H 19/207**, C07H 21/02

(21) Anmeldenummer: **87107482.9**

(22) Anmeldetag: **22.05.87**

## (54) Verfahren zur Herstellung von N6-substituierten NAD, NADP oder FAD.

(30) Priorität: **24.05.86 DE 3617535**

(43) Veröffentlichungstag der Anmeldung:
**02.12.87 Patentblatt 87/49**

(45) Bekanntmachung des Hinweises auf die Patenterteilung:
**04.09.91 Patentblatt 91/36**

(84) Benannte Vertragsstaaten:
**BE CH DE FR GB LI NL SE**

(56) Entgegenhaltungen:
**DE-A- 2 841 414**

**CHEMICAL ABSTRACTS, Band 108, 1988, Seite 381, Nr. 52347m, Columbus, Ohio, US; A.F. BUECKMANN: "A new synthesis of coenzymically active watersoluble macromolecular NAD and NADP derivatives", & BIOCATALYSIS 1987, 1(2), 173-86**

(73) Patentinhaber: **Gesellschaft für Biotechnologische Forschung mbH (GBF)
Mascheroder Weg 1
W-3300 Braunschweig-Stöckheim(DE)**

Patentinhaber: **Degussa AG
Wolfgang Rodenbacher Chaussee 4
W-6450 Hanau 1(DE)**

(72) Erfinder: **Bückmann, Andreas F.
Romintenstrasse 23
W-3300 Braunschweig(DE)**

(74) Vertreter: **Boeters, Hans Dietrich, Dr. et al
Boeters & Bauer Bereiteranger 15
W-8000 München 90(DE)**

**EP 0 247 537 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

An feste polymere Träger oder an wasserlösliche Makromoleküle gebundenes NAD(H) oder NADP(H) sind bereits seit etwa 15 Jahren untersucht und verwendet worden. Die an feste Träger gebundenen Coenzyme werden mit Erfolg in der Affinitätschromatcgraphie (1) eingesetzt.
Wasserlösliches makromolekulares NAD(H) oder NADP(H) wird als enzymatisch regenerierbares Coenzym-Derivat in Enzym-MembranReaktoren für die kontinuierliche Herstellung von Feinchemikalien durch Coenzym-abhängige enzymatische Katalyse (2) verwendet.

Eine herkömmliche Strategie zur Synthese dieser makromolekularen NAD(H)- oder NADP(H)-Derivate ist: Alkylierung der N(1)-Position des Adeninringsystems von NAD oder NADP, chemische Reduktion zu N-(1)-alkyliertem NADH oder NADPH, Dimroth-Umlagerung im basischen Milieu und bei erhöhten Temperaturen (pH 10-11, 60-70 °C) zu $N^6$-alkyliertem NADH oder NADPH, kovalente Bindung dieses reduzierten Coenzyms am Makromolekül oder enzymatische Oxidation zum $N^6$-alkylierten NAD oder NADP und anschließende kovalente Verknüpfung am Makromolekül.

Für NAD und NADP ist die Reaktionsfolge verwendet worden im Falle der Alkylierungsmittel wie Jodessigsäure (3,4), Propionlakton (5) 3,4-Epoxybuttersäure (6,7) oder Ethylenimin (8,9). Auf diese Weise alkylierte Coenzyme weisen für die kovalente Bindung an Makromolekülen Carboxyl- oder Aminogruppen am Ende der Kette am $N^6$-Atom des Adeninringes auf.

Für die Verknüpfung kommen solche unlöslichen Makromoleküle (feste Träger, Matrices) oder lösliche, insbesondere wasserlösliche, Makromoleküle in Betracht, die über eine oder mehrere für die Verknüpfung wirksamen funktionellen Gruppen verfügen. Die Makromoleküle können per se derartige funktionelle Gruppen aufweisen, bzw. die funktionellen Gruppen können in die Makromoleküle durch dem Fachmann bekannte Methoden eingeführt werden. Beispiele für verwendbare Makromoleküle sind:
Dextrane, Polyethylenglykole, Polyethylenimine, Polyacrylamide, Mischpolymere wie Methylvinylether/Maleinsäureanhydrid, Ethylen/ Maleinsäureanhydrid, Divinylether/Maleinsäureanhydrid, Agarose, Glas, Zellulose, Silicagel und Derivate der Makromoleküle. Für die Synthese makromolekularer Coenzymderivate sind alternative Strategien entwickelt worden, die als Varianten der herkömmlichen Strategie zu betrachten sind und manchmal eine Vereinfachung der Synthese bezwecken.
So ist eine durch Transglutaminase katalysierte kovalente Kopplung beschrieben worden im Falle der Bindung des herkömmlich synthetisierten $N^6$-[(6-aminohexyl)carbamoylmethyl]-$NAD^+$ an wasserlösliche Proteine, z. B. Casein via $\gamma$-Carboxyamidgruppen des L-Glutamins (10).
Auch ist es möglich, erst $N^6$-Vinyl-Derivate von NAD auf herkömmliche Weise herzustellen und diese mit anderen Vinylmonomeren zu makromolekularem NAD zu copolymerisieren (5). Ein vereinfachtes Copolymerisationsverfahren (Herstellung N(1)-Vinyl-$NAD^+$-Derivat und simultane Copolymerisation und Dimroth-Umlagerung) ergab ein makromolekulares NAD-Derivat, das nur beschränkt (<40 %) enzymatisch zu reduzieren war (11). Basierend auf wasserlöslichen Polymeren mit Epoxygruppen ist ein Verfahren bekannt, bei dem - ausgehend von unmodifiziertem NAD - die N(1)-Alkylierung, Reduktion zu N(1)-alkyliertem NADH und Dimroth-Umlagerung zu $N^6$-alkyliertemNADH in kovalent gebundenem Zustand am Polymer in einem Dreischritt-Verfahren durchgeführt wurde (12). Ausgehend von NADH ist sogar ein Einschrittverfahren möglich, da Kopplung durch N(1)-Alkylierung und Dimroth-Umlagerung im basischen Milieu unter gleichen Bedingungen simultan stattfinden können (12). Im besten Fall wurde makromolekulares NADH erhalten, das etwa 60 % enzymatisch oxidierbar war.
Diese vereinfachten Verfahren haben den Nachteil, daß nicht gut definierte makromolekulare NAD(H)-Derivate wegen Nebenreaktionen am Coenzym entstehen, die Hauptursache sind für die beschränkte Oxidier- oder Reduzierbarkeit.
Ein Kompromiß zwischen dem vereinfachten und herkömmlichen Verfahren mit dem Vorteil der Uniformität des gekoppelten NADH-Analogen konnte mit dem folgenden Verfahren erreicht werden: Synthese des N(1)-(2-Aminoethyl)-NAD, kovalente Bindung an ein wasserlösliches Polymer zu makromolekularem N(1)-(2-Aminoethyl)-NAD, chemische Reduktion zu makromolekularem N(1)-(2-Aminoethyl)-NADH und Dimroth-Umlagerung zu makromolekularem $N^6$-(2-Aminoethyl)-NADH(13, 14).

Es sei betont, daß alle Methoden zur Herstellung von makromolekularem NAD (H) oder NADP (H) mit gut definierten Coenzymen den Schritt der Dimroth-Umlagerung des reduzierten N(1)-alkylierten Coenzyms gemeinsam haben; vgl. beispielsweise DE-B-2 841 414. Diese Umlagerung findet für das Coenzym unter extremen Bedingungen statt, wodurch erhebliche Verluste im ungekoppelten Zustand auftreten können (pH 10,5 bis 11,2, Temperatur 60 bis 70 °C, Zeit 1,5 bis 2 h).

Für N(1)-(Carboxymethyl)-ATP konnten die Bedingungen der Dimroth-Umlagerung zu $N^6$-(Carboxymethyl)-ATP verbessert werden durch Zugabe eines Anionenaustauschers in $OH^-$-Form in der wäßrigen N(1)-(Carboxymethyl)-ATP-Lösung zur angeblichen Stabilisierung der Triphosphatgruppe (15).

Durch HPLC konnte die komplette Dimroth-Umlagerung mit stark reduziertem Produktzerfall innerhalb 5, bzw. 10 oder 240 Minuten während der Inkubation bei 100, 75 oder 50 °C nachgewiesen werden. Ausgehend von N(1)-(Carboxymethyl)-NAD konnte bei einem äquivalenten Vorgang kein coenzymatisch aktives $N^6$-(Carboxymethyl)-NAD hergestellt werden (15).

Gemäß einer Ausführungsform betrifft die Erfindung ein Verfahren zur Herstellung von $N^6$-substituiertem NAD oder NADP, bei dem man

(a) NAD oder NADP mit Ethylenimin in N(1)-Stellung zu N(1)-(2-Aminoethyl)-NAD oder N(1)-(2-Aminoethyl)-NADP alkyliert,

(b) das Alkylierungsprodukt einer Dimroth-Umlagerung zu $N^6$-(2-Aminoethyl)-NAD oder $N^6$-(2-Aminoethyl)-NADP unterwirft und

(c) gegebenenfalls das gemäß (a) oder (b) erhaltene Produkt in an sich bekannter Weise kovalent an ein Makromolekül bindet, wobei das Verfahren dadurch gekennzeichnet ist, daß man

(d) in wässerigem Milieu umlagert,

(e) dabei einen pH-Wert von 4 bis 9 und insbesondere 5 bis 8 einhält und

(f) eine Temperatur von 25 bis 75 °C und insbesondere von 40 bis 60 °C einhält,

(g) der Dimroth-Umlagerung keine Reduktion vorausgehen und entsprechend keine Oxidation folgen läßt und

(h) das bei der Stufe (b) als Nebenprodukt gebildete 1,$N^6$-Aethanadenin-NAD bzw. 1,$N^6$-Aethanadenin-NADP abtrennt und isoliert.

Beispielsweise kann man unter den angegebenen Bedingungen bei einer Temperatur von 50 °C 6 bis 8 h lang arbeiten.

FAD-Derivate, synthetisiert durch Modifizierung der $N^6$-Position des Adeninringes, sind bisher in zwei Fällen beschrieben worden in Zusammenhang mit der Immobilisierung an wasserlöslichen Polymeren oder festen Trägern.

Zapelli et al. (16) koppelten $N^6$-(2-Hydroxy-3-carboxypropyl)-FAD an Polyethylenimin, synthetisiert durch Dimroth-Umlagerung unter extremen Bedingungen (pH 10, 80 °C) des N(1)-(2-Hydroxy-3-carboxypropyl)-FAD nach Alkylierung des FAD mit 3,4-Epoxybuttersäure. Sie zeigten, daß für D-Aminosäureoxidase und Glucoseoxidase, die nicht-kovalent gebundenes und deshalb ausblutbares FAD besitzen, die Langzeitstabilität in immobilisierter Form in Anwesenheit des Polyethylenimin-$N^6$-(2-Hydroxy-Carboxypropyl)-FAD - funktionierend als ständiger FAD-Vorrat - stark verbessert werden konnte.

Narasimhan und Wingard (17) immobilisierten auf Indium/Zinnoxid-Elektroden FAD, das mit anwesenden Formaldehyd-aktivierten Aminosilangruppen reagierte, wodurch direkt eine $-NH-CH_2-NH-$Bindung zustande kam über die $N^6$-Position des Adeninringes. Dadurch konnten sie das Überpotential des NADH um 180 mV sinken, obwohl dies nicht genügte, um an der Elektrode befriedigend NADH in NAD zu oxidieren. Die Autoren erwähnen die Wahrscheinlichkeit, daß durch Nebenreaktionen auoh andere Kopplungsstellen am FAD-Molekül involviert sein könnten bei der Immobilisierung.

$N^6$-aminoalkylierte FAD-Derivate, beispielsweise $N^6$-(2-Aminoethyl)-FAD, sind bisher nicht beschrieben worden.

Gemäß einer weiteren Ausführungsform betrifft die Erfindung nun ein Verfahren zur Herstellung von $N^6$-substituiertem FAD, bei dem

(a) FAD in N(1)-Stellung alkyliert,

(b) das Alkylierungsprodukt ohne vorhergehende Reduktion und anschließende Reoxidation einer Dimroth-Umlagerung zum $N^6$-Derivat unterwirft,

(c) gegebenenfalls das gemäß (a) oder (b) erhaltene Produkt in an sich bekannter Weise kovalent an ein Makromolekül bindet, wobei das Verfahren dadurch gekennzeichnet ist, daß man

(d) die Alkylierung mit Ethylenimin durchführt und das so erhaltene N(1)-(2-Aminoethyl)-FAD in wässerigem Milieu zum $N^6$-(2-Aminoethyl)-FAD umlagert,

(e) dabei einen pH-Wert von 4 bis 9 und insbesondere von 5 bis 8 einhält und

(f) eine Temperatur von 25 bis 75 °C und insbesondere von 40 bis 60 °C einhält und

(g) das bei der Stufe (b) als Nebenprodukt gebildete 1,$N^6$-Aethanadenin-FAD abtrennt und isoliert.

Herstellung von makromolekularem NAD

A. Herstellung von N(1)-(2-Aminoäthyl)-NAD

Es wurden 200 g (300 mMol) NAD (Oriental yeast, freie Säure) in 400 ml destilliertem Wasser gelöst. Dazu wurden 42.5 ml (850 mMol) Ethylenimin (Serva) langsam zugegeben, wobei der pH-Wert bei 3.2 ± 0.05 mit 70 %iger Perchlorsäure gehalten wurde (Gesamtvolumen 650 ml). Die Reaktionsmischung wurde

50 Std. lang bei 30°C und pH 3.2 ± 0.05 gerührt (Einstellung mit 70 %iger Perchlorsäure). Die Umwandlung des NAD in N(1)-(2-Aminoethyl)-NAD wurde durch UV-Scanning nach Dünnschichtchromatographie auf Silicagel (DC-Alufolien Kieselgel 60 $F_{254}$, Schichtdicke 0.2 mm, E. Merck) mit Laufmittel Isobuttersäure/destill. Wasser/25 %iger $NH_3$ in Wasser 66/33/1 (Vol./Vol./Vol.), pH 3.7, ermittelt. Die Reaktionsmischung wurde mit destilliertem Wasser auf 1 Liter gebracht. Durch 5fach wiederholte Fällung und anschließende Zentrifugation mit jeweils der 10fachen Menge techn. Ethanol bei 4°C wurde das in Ethanol lösliche, nicht umgesetzte Ethylenimin entfernt. Der Niederschlag wurde unter Vakuum bei 25°C in einem Trockenschrank getrocknet und in einem Exsikkator über NaOH bei 4°C aufbewahrt.

Zur Fraktionierung wurden 20 g des trockenen Reaktionsgemisches in der Zusammensetzung: 5.3 mMol NAD (23 %), 15 mMol N(1)-(2-Aminoethyl)-NAD (65 %) und 2.6 mMol Nebenprodukte (12 %) in destilliertem Wasser gelöst bis zu 40 ml. Nach pH-Einstellung auf 5.0 mit 10 N NaOH wurde die Lösung bei 4°C auf eine Kationen-Austauschsäule gegeben (100 x 2,6 cm, Biorex 70, 50-100 Maschen, Bio-Rad), die gegen 0.01 M LiCl, pH 4.5, ins Gleichgewicht gebracht worden war. Nach dem Beschicken wurde das NAD in einer 2 Liter-Fraktion quantitativ mit 0.01 M LiCl, pH 4.5, eluiert (5.3 mMol). Weitere Elution mit 2 1 0.01 M LiCl, pH 4.5, ergab 2.5 mMol eines Gemisches mit zwei Verbindungen, die sich dünnschichtchromatographisch verhielten wie $N^6$-(2-Aminoethyl)-NAD und das vermutliche 1,$N^6$-Ethanadenin-NAD, die - wie unter B beschrieben - leicht aus N(1)-(2-Aminoethyl)-NAD entstehen können.

Die Säule wurde jetzt verkürzt auf 50 cm Länge durch Absenkung der Säulenfüllung, um eine schnellere Elution des Hauptproduktes N(1)-(2-Aminoethyl)-NAD zu erreichen. Diese Elution wurde mit 1.5 1 0.2 M LiCl, pH 4.7, durchgeführt und 11.3 mMol reines N(1)-(2-Aminoäthyl)-NAD gewonnen. Nebenprodukte (2.6 mhol), entstanden während der Alkylierung durch Ethylenimin, wurden nicht isoliert.

Die verschiedenen Fraktionen wurden unter reduziertem Druck (flash evaporation) auf 30 ml konzentriert und 5mal mit techn. Ethanol (20facher Überschuß auf Volumenbasis) gefällt, wodurch das in Ethanol gut lösliche Licl abgetrennt wird. Die Produkte der Fraktionen wurden nach Lösung in so wenig destilliertem Wasser wie möglich gefriergetrocknet und bei 4°C in einem Exsikkator über NaOH gelagert.

Die erzielte Fraktionierung ist in folgender Tabelle zusammen

| Verbindung | mMol im Reaktionsgemisch | mMol nach Fraktionierung | % Zurückgewinnung | % von mMol auf Säule gebracht |
|---|---|---|---|---|
| NAD | 5.3 | 5.3 | 100 | 23 |
| N(1)-(2-Aminoethyl)-NAD | 15 | 11.3 | 75 | 49 |
| Nebenprodukte, entstanden während Alkylierung | 2.6 | - | - | - |
| Nebenprodukte, entstanden auf Biorex 70-Säule aus N(1)-(2-Aminoethyl)-NAD | - | 2.5 | 16.6*) | 11 |

*) % bezogen auf N(1)-(2-Aminoethyl)-NAD in Reaktionsmischung

N(1)-(2-Aminoethyl)-NAD gibt mit Ninhydrin eine positive Reaktion (primäre Aminogruppen sind vorhanden) und zeigt eine für N(1)-Alkylierung im Adeninring charakteristische Schulter im Bereich von 300-310 nm bei pH 11.5.

gestellt:

B. Herstellung und Reinigung von $N^6$-(2-Aminoethyl)-NAD

Es wurden 2 g (278 mMol) N(1)-(2-Aminoethyl)-NAD in 200 ml destilliertem Wasser gelöst und mit 1 N LiOH auf pH 6.5 eingestellt. Diese Lösung wurde in einem Wasserbad bei 50 °C über 7 Stunden inkubiert. Jede Stunde erfolgte pH-Einstellung mit 1 N LiOH. Nach Dünnschichtchromatographie gemäß A wurde wahrgenommen, daß zwei Verbindungen aus N(1)-(2-Aminoethyl)-NAD entstanden waren, und zwar $N^6$-(2-Aminoethyl)-NAD (rf - 0.13) und das vermutliche 1,$N^6$-Ethanadenin-NAD (rf - 0.068). Die Zusammensetzung der Inkubationslösung wurde durch UV-Scanning nach Dünnschichtchromatographie bestimmt: 62.5 % (1.74 mMol) $N^6$-(2-Aminoethyl)-NAD und 37.5 % (1.04 mMol) 1,$N^6$-Ethanadenin-NAD.

Das gefriergetrocknete Reaktionsgemisch wurde in 16.5 ml destilliertem Wasser gelöst und mit 1 N LiOH auf pH 5.5 eingestellt. Diese Lösung wurde auf eine Kationen-Austauschersäule gegeben (100 x 1.6 cm, Biorex 70, 50-100 Maschen, Bio-Rad), die gegen 0.01 M LiCl, pH 3.5, ins Gleichgewicht gebracht worden war. Durch Elution bei 4 °C mit 0.01 M LiCl, pH 3.5, wurden beide Verbindungen getrennt eluiert mit einer leichten Überlappung, wobei $N^6$-(2-Aminoethyl)-NAD als letztes von der Säule kam.

Drei dünnschichtchromatographisch einheitliche Fraktionen mit 1,$N^6$-Ethanadenin-NAD (0.98 mMol in 250 ml), Gemisch (0.20 mMol in 120 ml) und reines $N^6$-(2-Aminoethyl)-NAD (1.56 mhol in 660 ml) wurden unter reduziertem Druck auf 3 ml konzentriert und zweimal mit kaltem techn. Äthanol (20facher Überschuß auf Volumenbasis) zur Entfernung des LiCl gefällt. Nach Aufnahme in so wenig destilliertem Wasser wie möglich wurden die Fraktionen gefriergetrocknet und bei 4 °C in einem Exsikkator über NaOH gelagert. Die erzielte Reinigung ist in folgender Tabelle zusammengestellt.

| Verbindung | mMol in Inkubationslösung | mMol nach Fraktionierung | % Zurückgewinnung | % von mMol auf Säule gebracht |
|---|---|---|---|---|
| 1,$N^6$-(Ethanadenin)-NAD | 1.04 | 0.98 | 94.2 | 30.8 |
| Gemisch | - | 0.21 | - | 7.7 |
| $N^6$-(2-Aminoethyl)-NAD | 1.73 | 1.56 | 90 | 56.5 |

UV-Spektren und NMR-Daten haben $N^6$-(2-Aminoethyl)-NAD als solches bestätigt. Die Verbindung gibt mit Ninhydrin eine positive Reaktion (primäre Aminogruppe anwesend). $\lambda_{max}$ liegt bei 267 nm, die Verbindung zeigt keine Schulter mehr im Bereich 300-310 nm bei pH 11.5, was charakteristisch wäre für N-(1)-Alkylierung des Adeninringes.

Quantitative Reduktion, katalysiert durch Bierhefe-Alkoholdehydrogenase, ergibt fluorescierendes $N^6$-(2-Aminoethyl-NADH (Excitation bei 366 nm) mit Extinktion bei 267 nm/Extinktion bei 338 nm = 3.2, charakteristisch für $N^6$-alkylierte NADH-Derivate.

C. Beispiel 1: Herstellung von Polyethylenglykol-$N^6$-(2-Aminoethyl)-NAD

2 g carboxyliertes Polyethylenglykol (M 20 000, 0.1 mMol mit 0.14 mMol Carboxylgruppen), hergestellt gemäß Bückmann et al. (Makromol, Chem. 182, 1379-1384 /1981/), wurden in destilliertem Wasser bis zu 4 ml gelöst und 2 ml mit 125 µMol $N^6$-(2-Aminoethyl)-NAD zugegeben. Nach pH-Einstellung auf 4.7 mit 1 N HCl wurden 200 mg (1.04 mMol) 1-(3-Dimethylaminopropyl)-3-Ethyl-carbodiimid-HCl in zwei gleichen Portionen innerhalb 10 Minuten zugegeben. Die Reaktionsmischung wurde 2 Stunden bei Raumtemperatur gerührt (pH-Einstellung im Bereich 4.5-4.8 mit 1 N HCl oder 1 N NaOH). Nach Inkubation während 16 Stunden bei 4 °C wurde das Reaktionsgemisch (7 ml) in zwei Chargen von 3.5 ml gelfiltriert über eine Sephadex G 50-Säule (100 x 2.6 cm), ins Gleichgewicht gegen destilliertes Wasser. Die vereinigten Fraktionen mit Polyethylenglykol-$N^6$-(2-Aminoethyl)-NAD wurden unter reduziertem Druck auf 15 ml konzentriert mit 85 µMol an Polyethylenglykol gebundenem $N^6$-(2-Aminoethyl)-NAD [Konzentration 5.5 mM] mit Kopplungsausbeute 68 %.

D. Beispiel 2: Herstellung von Dextran-$N^6$-(2-Aminoethyl)-NAD

0.2 g carboxyliertes Dextran T 70 (M 70 000, 1 mMol Anhydroglucosemonomere, wovon 0.3 mMol

carboxyliert waren), hergestellt gemäß Bückmann et al., J. Appl. Biochem. 3, 301-315 (1981), wurden in 2 ml destilliertem Wasser gelöst und 1 ml mit 65 µMol $N^6$-(2-Aminoethyl)-NAD zugegeben. Nach pH-Einstellung auf 4.7 mit 1 N HCl wurden 150 mg (0.78 mMol) 1-(3-Dimethylaminopropyl)-3-Ethyl-carbodiimid-HCl in zwei gleichen Portionen innerhalb 10 Minuten zugegeben. Die Reaktionsmischung wurde 2 Stunden bei Raumtemperatur gerührt (pH-Einstellung im Bereich 4.5-4.8 mit 1 N HCl oder 1 N NaOH). Nach Inkubation bei 4° C während 16 Stunden wurde das Reaktionsgemisch (3.5 ml) bei 4° C gelfiltriert über eine Sephadex G50-Säule (100 x 2.6 cm), ins gleichgewicht gegen destilliertes Wasser. Die Fraktion mit Dextran-$N^6$-(2-Aminoethyl)-NAD wurde unter reduziertem Druck zu 10 ml konzentriert mit 14 µMol an Dextran gebundenem $N^6$-(2-Aminoethyl)-NAD [Konzentration 1.4 mM] mit Kopplungsausbeute 22 %.

E. Beispiel 3: Herstellung von Polyvinylpyrrolidon-$N^6$-(2-Aminoethyl)-NAD

0.3 g carboxyliertes Polyvinylpyrrolidon ($\overline{M}$ 160 000, 2.7 mMol Vinylpyrrolidonmonomere mit 0.135 mMol Carboxylgruppen), hergestellt gemäß Specht, Hoppe-Seyler's Z. Physiol. Chem. 354, 1659-1660 (1973), wurden in 3 ml destilliertem Wasser gelöst, 100 mg (63 µMol) $N^6$-(2-Aminoethyl)-NAD zugegeben und der pH-Wert auf 4.7 eingestellt mit 1 N HCl. 150 mg (0.78 mMol) 1-(3-Dimethylaminopropyl)-3-Ethyl-carbodiimid-HCl wurden in zwei gleichen Portionen innerhalb 10 Minuten zugegeben. Die Reaktionsmischung wurde 2 Stunden bei Raumtemperatur gerührt (pH-Einstellung im Bereich 4.5-4.8 mit 1 N HCl oder 1 N NaOH). Nach Inkubation bei 4° C während 16 Stunden wurde das Reaktionsgemisch (3.8 ml) bei 4° C gelfiltriert über eine Sephadex G 50-Säule (100 x 2.6 cm), ins Gleichgewicht gegen destilliertes Wasser. Die Fraktion mit Polyvinylpyrrolidon-$N^6$-(2-Aminoethyl)-NAD wurde konzentriert unter reduziertem Druck zu 10 ml mit 10 µMol an Polyvinylpyrrolidon gebundenem $N^6$-(2-Aminoethyl)-NAD [Konzentration 1 mM) mit Kopplungsausbeute 16 %.

F. Beispiel 4: Herstellung von Poly-(Ethylen/Maleinsäure)-$N^6$-(2-Aminoethyl)-NAD

50 mg Poly-(Ethylen/Maleinsäureanhydrid) ($\overline{M}$ unbekannt, 0.4 mMol Ethylen/Maleinsäureanhydridmonomere, Aldrich) wurden in 10 ml destilliertem Wasser gelöst (pH-Einstellung auf 7.5 mit 1 N NaOH) und 20 µMol $N^6$-(2-Aminoethyl)-NAD zugegeben. Nach 5 Stunden Rühren bei Raumtemperatur (pH-Einstellung auf 7.5 mit 1 N NaOH) wurde das Reaktionsgemisch gegen 5 l destilliertes Wasser bei 4° C während 16 Stunden dialysiert. 22 ml mit 13 µMol an Poly-(Ethylen/Maleinsäure) gebundenes $N^6$-(2-Aminoethyl)-NAD [Konzentration 0.6 mM] mit Kopplungsausbeute 65 % wurden erhalten.

G. Beispiel 5: Herstellung von Poly-(Methylvinylether/Maleinsäure)-$N^6$-(2-Aminoethyl)-NAD

50 g Poly-Methylvinylether/Maleinsäureanhydrid) ($\overline{M}$ 20 000, 0.32 mMol Methylvinylether/Maleinsäureanhydrid-monomere, Polysciences) wurden in 10 ml destilliertem Wasser (pH-Einstellung auf 7.5 mit 1N NaOH gelöst und 20 µMol $N^6$-(2-Aminoethyl)-NAD zugegeben. Nach 5 Stunden Rühren bei Raumtemperatur (pH-Einstellung auf 7.5 mit 1 N NaOH) wurde das Reaktionsgemisch gegen 5 l destilliertes Wasser bei 4° C während 16 Stunden dialysiert. 22 ml mit 17 µMol an Poly-(Methylvinylether/Maleinsäure) gebundenes $N^6$-(2-Aminoethyl)-NAD [Konzentration 0.78 mM] mit Kopplungsausbeute 85 % wurden erhalten.

H. Beispiel 6: Herstellung von Poly-(Divinylether/Maleinsäure)-$N^6$-(2-Aminoethyl)-NAD

50 mg Poly-(Divinylether/Maleinsäurenahydrid) ($\overline{M}$ 18 000, 0.19 mMol Divinylether/Maleinsäure-anhydridmonomere, Hercules) wurden in 10 ml destilliertem Wasser (pH-Einstellung auf 7.5 mit 1 N NaOH) gelöst und 20 µMol $N^6$-(2-Aminoethyl)-NAD zugegeben. Nach 5 Stunden Rühren bei Raumtemperatur (pH-Einstellung auf 7.5 mit 1 N NaOH) wurde das Reaktionsgemisch gegen 5 l destilliertes Wasser bei 4° C während 16 Stunden dialysiert. 22 ml mit 20 µMol an Poly-(Divinylether/Maleinsäure) gebundenes $N^6$-(2-Aminoethyl)-NAD [Konzentration 0.92 mM] mit Kopplungsausbeute 100 % wurden erhalten.

Die enzymatische Reduzierbarkeit der wasserlöslichen polymergebundenen $N^6$-(2-Aminoethyl)-NAD-Derivate wurde im Vergleich zu $N^6$-(2-Aminoethyl)-NAD bestimmt mit der von Hefe-Alkohol-Dehydrogenase katalysierten Reaktion unter folgenden Bedingungen:

0.1 M Tris/HCl, pH 8.2, Raumtemperatur
0.1 M Ethanol
7 mM Semicarbazid/HCl
0.1 M $N^6$-(2-Aminoethyl)-NAD

(frei oder polymer-gebunden)
0.3 mg Alkoholdehydrogenase/ml

Die Reduzierbarkeitsdaten sind in folgender Tabelle zusammengestellt:

| | Reduzierbarkeit % |
|---|---|
| $N^6$-(2-Aminoethyl)-NAD | 100 |
| Polyethylenglykol (M 20 000)-$N^6$-(2-Aminoethyl)-NAD | 90 |
| Dextran T 70 (M 70 000)-$N^6$-(2-Aminoethyl)-NAD | 90 |
| Polyvinylpyrrolidon (M 160 000)-$N^6$-(2-Aminoethyl)-NAD | 90 |
| Poly-(Ethylen/Maleinsäure)-$N^6$-(2-Aminoethyl)-NAD | 60 |
| Poly-(Methylvinylether/Maleinsäure)-$N^6$-(2-Aminoethyl)-NAD | 70 |
| Poly-(Divinylether/Maleinsäure)-$N^6$-(2-Aminoethyl)-NAD | 60 |

N.B.: Für die $N^6$-(2-Aminoethyl)-NAD- und $N^6$-(2-Aminoethyl)-NADH-Derivate wurden $\epsilon_{267} = 21\,000\ M^{-1}cm^{-1}$ und $\epsilon_{340} = 6220\ M^{-1}cm^{-1}$ als Extinktionskoeffizienten angenommen.

I. Beispiel 7: Herstellung von CH-Sepharose 4B-$N^6$-(2-Aminoethyl)-NAD

1.5 g CH-Sepharose 4B (Pharmacia, mit 60-84 µMol Carboxyhexylgruppen) gequollen in 10 ml destilliertem Wasser, wurden über einer Glasfritte mit 100 ml destilliertem Wasser gewaschen. Das halbtrockene CH-Sepharose 4B wurde suspendiert in 2 ml destilliertem Wasser und 430 mg (2.25 mMol) 1-(3-Dimethylaminopropyl)-3-Ethyl-Carbodiimid-HCl zugegeben. Die Suspension wurde 10 min inkubiert bei Zimmertemperatur (pH-Einstellung auf 4.8-5.0 mit 1 N NaOH oder 1 N HCl). Das aktivierte CH-Sepharose 4B wurde innerhalb einer Minute zweimal mit 25 ml destilliertem Wasser gewaschen und addiert an 2 ml wäßriger Lösung mit 34 µMol $N^6$-(2-Aminoethyl)-NAD. Die Suspension wurde 16 Stunden bei Zimmertemperatur inkubiert nach pH-Einstellung auf 4.6 (1 N NaOH oder 1 N HCl).

Nach Waschen mit 75 ml 20 % LiCl und 25 ml destilliertem Wasser und Sedimentation in destilliertem Wasser wurden 7 ml CH-Sepharose 4B-$N^6$-(2-Aminoethyl)-NAD mit 20.6 µMol gebundenem $N^6$-(2-Aminoethyl)-NAD [Konzentration 3 mM] erhalten mit Kopplungsausbeute 60.5 %.

Herstellung von makromolekularem NADP

A. Herstellung und Reinigung von N(1)-(2-Aminoethyl)-NADP

Es wurden 7.5 g (9.51 mMol) NADP (Boehringer, Di-Natriumsalz) in 10 ml destilliertem Wasser gelöst. Dazu wurden 1.4 ml (28 mMol) Ethylenimin (Serva) langsam zugegeben, wobei der pH-Wert bei 3.35 ± 0.05 mit 70%iger Perchlorsäure gehalten wurde (Gesamtvolumen 25 ml). Die Reaktionsmischung wurde 120 Stunden lang bei 30 °C, pH 3.35 ± 0.05, gerührt (Einstellung mit 70 %iger Perchlorsäure). Die Umwandlung des NADP in N(1)-(2-Aminoethyl)-NADP wurde ermittelt durch UV-Scanning nach Dünnschichtchromatographie auf Silicagel (DC-Alufolien Kieselgel 60 $F_{254}$, Schichtdicke 0.2 mm, E. Merck) mit Laufmittel Isobuttersäure/destilliertes Wasser/25 %iger $NH_3$ in Wasser = 66/33/1 (Vol./Vol./Vol.), pH 3.7. Die Reaktionsmischung wurde mit destilliertem Wasser auf 50 ml gebracht. Durch 5fach wiederholte Fällung und

anschließende Zentrifugation mit jeweils der 10fachen Menge techn. Ethanol bei 4° C wurde das in Ethanol lösliche, nicht umgesetzte Ethylenimin entfernt. Der Niederschlag wurde in so wenig destilliertem Wasser wie möglich aufgenommen und gefriergetrocknet. Das Lyophilisat wurde in einem Exsikkator über NaOH bei 4° C aufbewahrt.

Zur Fraktionierung wurden 0.75 g gefriergetrocknete Reaktionsmischung mit Zusammensetzung 0.235 mMol NADP (27.5 %), 0.575 mMol N(1)-(2-Aminoethyl)-NADP (68 %) und 0.038 mMol Nebenprodukte (4.5 %) in destilliertem Wasser gelöst bis zu 22 ml. Nach pH-Einstellung auf 3.5 mit 1 N HCl wurde diese Lösung auf eine Kationen-Austauschersäule gegeben (60 x 1.5 cm, AG W50 $X_4$, 100-200 Maschen, Bio-Rad), die gegen 0.01 M Triethanolamin-Bicarbonat, pH 3.5, ins Gleichgewicht gebracht worden war. Durch Elution bei 4° C mit 0.01 M Triethanolamin-Bicarbonat, pH 3.5, wurden sukzessive drei Fraktionen gewonnen: mit Nebenprodukten verunreinigtes NADP (0.29 mMol, 34 % in 25 ml), Nebenprodukte (0.115 mMol, 13.5 % in 100 ml), entstanden während der Alkylierungsreaktion aus N(1)-(2-Aminoethyl)-NADP und während der Fraktionierung auf der Säule, und reines N(1)-(2-Aminoethyl)-NADP (0.39 mMol, 46 %, in 200 ml).

Die Fraktionen wurden unter reduziertem Druck bis 25 ml konzentriert und gefriergetrocknet zur Entfernung des Triethanolamin-Bicarbonats und bei 4° C in einem Exsikkator über NaOH gelagert. Die erzielte Fraktionierung ist in der folgenden Tabelle zusam

| Verbindung | mMol im Reaktionsgemisch | mMol nach Fraktionierung | % Zurückgewinnung | % von mMol auf Säule gebracht |
|---|---|---|---|---|
| NADP | 0.235 | 0.235 | 100 | 27.6 |
| Nebenprodukte | 0.038 | 0.17 | - | 20 |
| N(1)-(2-Amino-ethyl)-NADP | 0.575 | 0.39 | 68 | 49 |

mengestellt. N(1)-(2-Aminoethyl)-NADP gibt mit Ninhydrin eine positive Reaktion (primäre Aminogruppen sind vorhanden) und zeigt eine für N(1)-Alkylierung im Adeninring charakteristische Schulter im Bereich 300-310 nm bei pH 11.5.

B. Herstellung und Reinigung von $N^6$-(2-Aminoethyl)-NADP

Es wurden 0.35 g (0.24 mMol) N(1)-(2-Aminoethyl)-NADP gelöst in 0.5 l destilliertem Wasser und mit 1 N LiOH ein pH-Wert von 6.0 eingestellt. Diese Lösung wurde in einem Wasserbad bei 50° C während 4 Stunden inkubiert. Stündlich erfolgte pH-Einstellung mit 1 N LiOH. Durch Dünnschichtchromatographie gemäß A wurde wahrgenommen, daß zwei Verbindungen aus N(1)-(2-Aminoethyl)-NADP entstanden waren, und zwar $N^6$-(2-Aminoethyl)-NADP (rf = 0.048) und das vermutliche 1,$N^6$-Ethan-Adenin-NAD (rf = 0.031).
Die Zusammensetzung der Inkubationslösung wurde durch UV-scanning bei 260 nm nach Dünnschichtchromatographie bestimmt: 75 % 1,$N^6$-Ethan-Adenin-NAD (0.18 mMol) und 25 % $N^6$-(2-Aminoethyl)-NADP (0.06 mMol).
Die Inkubationslösung wurde unter reduziertem Druck auf 7 ml eingeengt. Nach pH-Einstellung auf 5.0 wurde diese Lösung bei 4° C auf eine Anionen-Austauschersäule gegeben (100 x 1.6 cm, $AG_1X_4$, 100-200 Maschen, Bio-Rad), die gegen 0.01 M Triethanolamin-Bicarbonat, pH 3.5, ins Gleichgewicht gebracht worden war. Durch Elution mit 0.01 M Triethanolimin-Bicarbonat, pH 3.5, wurde sukzessive in zwei Hauptfraktionen: 1,$N^6$-Ethan-Adenin-NAD (0.17 mMol, 70 % in 1 Liter) und $N^6$-(2-Aminoethyl)-NADP (0.053 mMol, 22 %, in 250 ml) eluiert.
Die zwei Fraktionen wurden unter reduziertem Druck auf 2 ml eingeengt und zur Entfernung des Triethanolamin-Biocarbonats zweimal bei Raumtemperatur über eine Sephadex G10-Säule (100 x 1.5 cm), die gegen destilliertes Wasser ins Gleichgewicht gebracht worden war, eluiert.
Nach Gefriertrocknung wurden beide Produkte in einem Exsikkator über NaOH bei 4° C gelagert. Die erzielte Fraktionierung ist in der folgenden Tabelle zusammengestellt:

| Verbindung | mMol in Inkubationslösung | mMol nach Fraktionierung | % Zurückgewinnung | % von mMol auf Säule gebracht |
|---|---|---|---|---|
| 1,$N^6$-Ethan-Adenin-NADP | 0.18 | 0.17 | 94.4 | 70.8 |
| $N^6$-(2-Aminoethyl)-NADP | 0.06 | 0.053 | 88.3 | 22.0 |

UV-Spektren und NMR-Daten haben $N^6$-(2-Aminoethyl)-NADP als solches bestätigt. Die Verbindung gibt mit Ninhydrin eine positive Reaktion (primäre Aminogruppe anwesend), hat $\lambda_{max}$ bei 267 nm und zeigt keine Schulter mehr im Bereich 300-310 nm bei pH 11.5, die für die N(1)-Alkylierung des Adeninringes charakteristisch wäre. Quantitative Reduktion, katalysiert durch Bierhefe-Glukose-6-Phosphatdehydrogenase, ergibt fluorescierendes $N^6$-(2-Aminoethyl)-NADPH (Excitation bei 366 nm) mit Extinktion bei 267 nm/Extinktion bei 338 = 3.2, charakteristisch für $N^6$-alkylierte NADPH-Derivate.

C. Beispiel 1: Herstellung von Polyethylenglykol-$N^6$-(2-Aminoethyl)-NADP

PEG ($M_r$ = 4000)-$N^6$-(2-Aminoethyl)-NADP:
60 mg N-Hydroxysuccinimid-aktiviertes, carboxyliertes Polyethylenglykol (M 4 000, 0.015 mMol mit 0.03 mMol aktivierte Carboxylgruppen), hergestellt gemäß Bückmann et al., Makromol. Chem. 182, 1379-1384 (1981), wurden in 1 ml destilliertem Wasser mit 15 µMol $N^6$-(2-Aminoethyl)NADP gelöst, bei pH 7.2, eingestellt mit 1 N NaOH. 5 Stunden wurde bei Raumtemperatur und pH 7.2 gerührt (pH-Einstellung mit 1 N NaOH). Das Reaktionsgemisch wurde bei 4° C über eine Sephadex G50-Säule (100 x 2.6 cm) gelfiltriert, ins Gleichgewicht gegen destilliertes Wasser. Die Fraktion mit Polyethylenglykol-$N^6$-(2-Aminoethyl)-NADP wurde eingeengt auf 10 ml mit 12 µMol an Polyethylenglykol ($M_r$ = 4000) gebundenem $N^6$-(2-Aminoethyl)-NADP [Konzentration 1.2 mM] mit Kopplungsausbeute 80 %.

PEG ($M_r$ = 20,000)-$N^6$-(2-Aminoethyl)-NADP:
325 mg N-Hydroxysuccinimid aktiviertes, carboxyliertes Polyethylenglykol ($M_r$ = 20,000, 0.015 mMol mit 0.03 mMol aktivierte Carboxylgruppen), hergestellt gemäß Bückmann et al., Makromol. Chem. 182, 1379-1384 (1981), wurden in 1 ml destilliertem Wasser mit 15 µMol $N^6$-(2-Aminoethyl)-NADP bei pH 7.2 gelöst, eingestellt mit 1 N NaOH. Bei Zimmertemperatur wurde während einer Stunde bei pH 7.2-7.3 gerührt und 3 Stunden bei pH 6.8 (1 N HCl oder 1 N NaOH). Das Reaktionsgemisch wurde gelfiltriert bei 4° C über eine Sephadex G 50-Säule (2.6 x 100 cm), ins Gleichgewicht gegen destilliertes Wasser. Die Fraktion mit Polyethylenglykol $N^6$-(2-Aminoethyl)-NADP wurde eingeengt auf 10 ml mit 15 µMol an Polyethylenglykol-($M_r$ = 20,000) gebundenem $N^6$-(2-Aminoethyl)-NADP [Konzentration 1.5 mM] mit Kopplungsausbeute 100 %.

NB: Die Kopplungsmethode, basierend auf Aktivierung der Carboxylgruppen mit 1-(3-Dimethylaminopropyl)-3-ethyl-carbodiimid, kommt für NADP-Derivate nicht infrage, weil dieses Reagenz auch die 2,3-Zyklisierung über der 2-Phosphatgruppe der Ribose an der Adeninseite des Moleküles bewirkt, wodurch NADP-Derivate die Coenzymaktivität verlieren.

D. Beispiel 2: Herstellung von Poly-(Ethylen/Maleinsäure)-$N^6$-(2-Aminoethyl)-NADP

12 mg Poly-(Ethylen/Maleinsäureanhydrid) (M unbekannt, 0.1 mMol Ethylen/Maleinsäureanhydrid-Monomere, Aldrich) wurden in 1 ml destilliertem Wasser (pH-Einstellung auf 7.5 mit 0.2 N NaOH) gelöst und 5.5 µMol $N^6$-(2-Aminoethyl)-NADP zugegeben. Nach 5 Stunden Rühren bei Raumtemperatur (pH-Einstellung auf 7.5 mit 0.2 N NaOH) wurde das Reaktionsgemisch gegen 5 Liter bei 4° C während 16 Stunden dialysiert. 3.3 ml mit 0.8 µMol an Poly-(Ethylen/Maleinsäure) gebundenem $N^6$-(2-Aminoethyl)-NADP [Konzentration 0.24 mM] mit Kopplungsausbeute 15 % wurden erhalten.

E. Beispiel 3: Herstellung von Poly-(Methylvinylether/Maleinsäure)-$N^6$-(2-Aminoethyl)-NADP

12 mg Mol-(Methylvinylether/Maleinsäureanhydrid) (M 20 000, 0.077 mMol Methylvinylether/Maleinsäureanhydrid-Monomere, Polysciences) wurden in 1 ml destilliertem Wasser (pH-Einstellung auf 7.5 mit 0.2 N NaOH) gelöst und 5.5 µMol $N^6$-(2-Aminoethyl)-NADP zugegeben. Nach 5

Stunden Rühren bei Raumtemperatur (pH-Einstellung auf 7.5 mit 0.2 N NaOH) wurde das Reaktionsgemisch gegen 5 Liter bei 4° C während 16 Stunden dialysiert. 4.4 ml mit 0.6 µMol an Poly-(Methylvinylether/Maleinsäure) gebundenem $N^6$-(2-Aminoethyl)-NADP [Konzentration 0.2 mM] mit Kopplungsausbeute 11 % wurden erhalten.

F. Beispiel 4: Herstellung von Poly-(Divinylether/Maleinsäure)-$N^6$-(2-Aminoethyl)-NADP

12 mg Poly-(Divinylether/Maleinsäureanhydrid) (M 18 000, 0.046 µMol Divinylether/Maleinsäureanhydrid-Monomere, Hercules) wurden in 1 ml destilliertem Wasser (pH-Einstellung auf 7.5 mit 0.2 N NaOH) gelöst und 5.5 µMol $N^6$-(2-Aminoethyl)-NADP zugegeben. Nach 5 Stunden Rühren bei Raumtemperatur (pH-Einstellung auf 7.5 mit 0.2 N NaOH) wurde das Reaktionsgemisch gegen 5 Liter bei 4° C während 16 Stunden dialysiert. 3.5 ml mit 0.7 µMol an Poly-(Divinylether/Maleinsäure) gebundenem $N^6$-(2-Aminoethyl)-NADP [Konzentration 0.136 mM] mit Kopplungsausbeute 13 % wurden erhalten.

NB: Die $N^6$-(2-Aminoethyl)-NADP-Kopplungsausbeute für D, E und F ist wesentlich niedriger im Vergleich zu ähnlichen Kopplungen im Falle $N^6$-(2-Aminoethyl)-NAD. Die aus dem Säureanhydrid entstehenden negativ geladenen Carboxylgruppen werden die Kopplung für die auch negativen $N^6$-(2-Aminoethyl)-NADP-Moleküle erschweren im Vergleich zu den positiv geladenen $N^6$-(2-Aminoethyl)-NAD-Molekülen.

Die enzymatische Reduzierbarkeit der wasserlöslichen Polymer-gebundenen $N^6$-(2-Aminoethyl)-NADP-Derivate wurde im Vergleich zu $N^6$-(2-Aminoethyl)-NADP bestimmt mit der von Hefe-Glukose-6-Phosphatdehydrogenase katalysierten Reaktion unter folgenden Bedingungen:

0.05 M Triethanolamin/HCl, pH 8.0, Raumtemperatur
5.5 mM $MgCl_2$
4.5 mM Glukose-6-Phosphat
0.1 mM $N^6$-(2-Aminoethyl)-NADP (frei oder Polymer-gebunden)
30 µg Glukose-6-Phosphatdehydrogenase

Die Reduzierbarkeitsdaten sind in folgender Tabelle zusammengestellt:

| | Reduzierbarkeit % |
|---|---|
| $N^6$-(2-Aminoethyl)-NADP | 100 |
| Polyethylenglykol(M 4000)-$N^6$-(2-Aminoethyl)-NADP | 95 |
| Polyethylenglykol(M 20,000)-$N^6$-(2-Aminoethyl)-NADP | 95 |
| Poly-(ethylen/Maleinsäure)-$N^6$-(2-Aminoethyl)-NADP | 50 |
| Poly-(Methylvinylether/Maleinsäure)-$N^6$-(2-Aminoethyl)-NADP | 60 |
| Poly-(Divinylether/Maleinsäure)-$N^6$-(2-Aminoethyl)-NADP | 63 |

NB: Für die $N^6$-(2-Aminoethyl)-NADP- und $N^6$-(2-Aminoethyl)-NADPH-Derivate wurden $\epsilon_{267} = 21\,000\ M^{-1}cm^{-1}$ und $\epsilon_{340} = 6220\ M^{-1}cm^{-1}$ als Extinktionskoeffizienten angenommen.

G. Beispiel 5: Herstellung von CH-Sepharose 4B-$N^6$-(2-Aminoethyl)-NADP:

0.25 g CH-Sepharose 4B (Pharmacia, mit 10-14 µMol Carboxyhexylgruppen) gequollen in 3 ml

destilliertem Wasser, wurden über einer Glasfritte mit 25 ml destilliertem Wasser gewaschen. Das halbtrokkene CH-Sepharose 4B wurde suspendiert in 0.45 ml destilliertem Wasser und 70 mg (365 µMol) 1-(3-Dimethylaminopropyl)-3-Ethyl-carbodiimid-HCl zugegeben. Die Suspension wurde 10 min später inkubiert bei Zimmertemperatur (pH-Einstellung auf 4.8-5.0 mit 0.1 N NaOH oder 0.1 N HCl). Das aktivierte CH-Sepharose 4B wurde innerhalb einer Minute zweimal mit 10 ml destilliertem Wasser gewaschen und addiert an 0.45 ml wäßriger Lösung mit 1.4 µMol $N^6$-(2-Aminoethyl)-NADP. Die Suspension wurde 16 Stunden bei Zimmertemperatur inkubiert nach pH-Einstellung auf 4.6 (0.1 N NaOH oder 0.1 N HCl).

Nach Waschen mit 20 ml 20 % LiCl und 16 ml destilliertem Wasser und Sedimentation in destilliertem Wasser wurden 1 ml CH-Sepharose 4B-$N^6$-(2-Aminoethyl)-NADP mit 1 µMol gebundenem $N^6$-(2-Aminoethyl)-NADP [Konzentration 1 mM] erhalten mit Kopplungsausbeute 71.4 %.

Herstellung von makromolekularem FAD

A. Herstellung und Reinigung von N(1)-(2-Aminoethyl)-FAD

Es wurden 0.5 g (0.6 mMol) FAD (Serva, Di-Natriumsalz) in 1 ml destilliertem Wasser gelöst. Dazu wurden 40 µl (0.8 mMol) Ethylenimin (Serva) langsam zugegeben, wobei der pH-Wert bei 3.5 ± 0.1 mit 35 %iger Perchlorsäure gehalten wurde (Gesamtvolumen 1.5 ml). Die Reaktionsmischung wurde 144 Stunden lang bei 30° C, pH 3.5 ± 0.1, gerührt (Einstellung mit 35 %iger Perchlorsäure). Die Umwandlung des FAD in N(1)-(2-Aminoethyl)-FAD wurde ermittelt durch UV-Scanning nach Dünnschichtchromatographie auf Silicagel (DC-Alufolien Kieselgel 60 $F_{254}$, Schichtdicke 0.2 mm, E. Merck) mit Laufmittel Isobuttersäure/destilliertes Wasser / 25 %iger $NH_3$ in Wasser = 66/33/1 (Vol./Vol./Vol.), pH 3.7. Die Reaktionsmischung wurde mit destilliertem Wasser auf 3 ml gebracht. Durch 2fach wiederholte Fällung und anschließende Zentrifugation mit jeweils 50 ml techn. Ethanol bei 4° C wurde das in Ethanol lösliche, nicht umgesetzte Ethylenimin entfernt. Der Niederschlag wurde in so wenig destilliertem Wasser wie möglich aufgenommen und gefriergetrocknet. Das Lyophilisat wurde in einem Exsikkator über NaOH bei 4° C aufbewahrt.

Zur Fraktionierung wurde die gefriergetrocknete Reaktionsmischung mit Zusammensetzung 0.192 mMol FAD (32 %), 0.354 mMol N(1)-(2-Aminoethyl)-FAD (59 %) und 0.054 mMol Nebenprodukte (9 %) in destilliertem Wasser gelöst bis zu 20 ml. Nach pH-Einstellung auf 5.0 mit 1 N HCl wurde diese Lösung auf eine Kationen-Austauschersäule gegeben (60 x 1.5 cm, Biorex 70, 50-100 Maschen, Bio-Rad), die gegen destilliertes Waser, pH 3.5, ins Gleichgewicht gebracht worden war. Durch Elution bei 4° C mit destilliertem Wasser, pH 3.5, wurden sukzessive zwei Fraktionen gewonnen: mit Nebenprodukten verunreinigtes FAD (0.22 mMol, 46.6 % in 150 ml) und Nebenprodukte (0.043 mMol, 8.9 % in 100 ml), entstanden während der Fraktionierung auf der Säule. Reines N(1)-(2-Aminoethyl)-FAD (0.215 mMol, 44.5 %, in 1000 ml) wurde eluiert mit 0.5 M LiCl, pH 3.5.

Die Fraktionen mit FAD und N(1)-(2-Aminoethyl)-FAD wurden unter reduziertem Druck bis 5 resp. 10 ml eingeengt. Aus der N(1)-(2-Aminoethyl)-FAD-Fraktion wurde durch 3fach weiderholte Fällung und anschließende Zentrifugation in jeweils 250 ml techn. Ethanol bei 4° C LiCl entfernt. Die FAD-Fraktion und N(1)-(2-Aminoethyl)-FAD wurden nach Lösen in 10 ml destilliertem Wasser gefriergetrocknet und bei 4° C einem Exsikkator über NaOH gelagert.

Die erzielte Fraktionierung ist in der folgenden Tabelle zusammengestellt:

| Verbindung | mMol im Reaktionsgemisch | mMol nach Fraktionierung | % Zurückgewinnung | % von mMol auf Säule gebracht |
|---|---|---|---|---|
| FAD | 0.192 | 0.192 | 100 | 32 |
| Nebenprodukte | 0.054 | 0.071 | - | 11.8 |
| N(1)-(2-Amino-ethyl)-FAD | 0.354 | 0.215 | 61 | 36 |

N(1)-(2-Aminoethyl)-FAD gibt mit Ninhydrin eine positive Reaktion (primäre Aminogruppen sind vorhanden) und zeigt die Maxima im UV-Spektrum, üblich für FAD und Derivate bei 450, 373 und 262 nm. Angeregt bei

360 nm wird starke Fluoreszenz beobachtet. Der rf-Wert im o.g. Elutionssystem für Dünnschicht-Chromatographie beträgt 0.138.

B. Herstellung und Reinigung von $N^6$-(2-Aminoethyl)-FAD

Es wurden 39 µMol N(1)-(2-Aminoethyl)-FAD gelöst in 8.3 ml destilliertem Wasser und mit 0.1 N LiOH ein pH-Wert von 6.5 eingestellt. Diese Lösung wurde in einem Wasserbad bei 40 °C während 7 Stunden inkubiert. Stündlich erfolgte pH-Einstellung mit 0.1 N LiOH. Durch Dünnschichtchromatographie gemäß A wurde wahrgenommen, daß zwei Verbindungen aus N(1)-(2-Aminoethyl)-FAD entstanden waren, und zwar $N^6$-(2-Aminoethyl)-FAD (rf = 0.177) und das vermutliche 1,$N^6$-Ethan-Adenin-FAD (rf = 0.12). Die Zusammensetzung der Inkubationslösung wurde durch UV-scanning bei 265 nm nach Dünnschichtchromatographie bestimmt: 35 % 1,$N^6$-Ethan-Adenin-FAD (13.5 µMol) und 65 % $N^6$-(2-Aminoethyl)-FAD (25.5 µMol).

Die Inkubationslösung wurde unter reduziertem Druck auf 4 ml eingeengt. Nach pH-Einstellung auf 6.5 wurde diese Lösung bei 4 °C auf eine Anionen-Austauschersäule gegeben (100 x 0.5 cm, $AG_1X_4$, 100-200 Maschen, Bio-Rad), die gegen destilliertes Wasser, pH 3.5, in Gleichgewicht gebracht worden war. Durch Elution mit destilliertem Wasser, pH 3.5, wurden erst undefinierte Zersetzungsprodukte eluiert (± 2 µMol, 5 %). Durch einen Gradienten 0 - 0.2 M LiCl (pH 3.5, 500 ml/500 ml) wurden sukzessive $N^6$-(2-Aminoethyl)-FAD (13.7 µMol, 53 %, in 400 ml) und das vermutliche 1,$N^6$-Ethan-Adenin-FAD (5.6 µMol, 40 %, in 300 ml) eluiert. Die zwei Fraktionen wurden unter reduziertem Druck auf 4 ml eingeengt und zur Entfernung des LiCl über eine Sephadex G10-Säule (100 x 1cm), die gegen destilliertes Wasser in Gleichgewicht gebracht worden war, gelfiltriert.

Nach Gefriertrocknung wurden beide Produkte in einem Exsikkator über NaOH bei 4 °C gelagert. Die erzielte Fraktionierung ist in der folgenden Tabelle zusammengestellt:

| Verbindung | µMol im Reaktionsgemisch | µMol nach Fraktionierung | % Zurückgewinnung | % von µMol auf Säule gebracht |
|---|---|---|---|---|
| 1.$N^6$-Ethan-Adenin-FAD | 13.5 | 5.5 | 40 | 14.3 |
| $N^6$-(2-Amino-ethyl-FAD | 25.5 | 13.7 | 53 | 35.1 |

UV-Spektren und erste NMR-Daten haben $N^6$-(2-Aminoethyl)-FAD als solches bestätigt. Die Verbindung gibt mit Ninhydrin eine positive Reaktion (primäre Aminogruppe anwesend), hat $\lambda_{max}$ bei 266 nm und zeigt die für FAD und Derivate charakteristischen Maxima bei 450 und 373 nm im UV-Spektrum. Angeregt bei 366 nm wird starke Fluorescenz wahrgenommen.

C. Beispiel 1: Herstellung von Polyethylenglykol-$N^6$-(2-Aminoethyl)-FAD

PEG ($M_r$ = 20,000)-$N^6$-(2-Aminoethyl)-FAD:
110 mg N-Hydroxysuccinimid aktiviertes, carboxyliertes Polyethylenglykol ($M_r$ = 20,000, 5 µMol mit 10 µMol aktivierte Carboxylgruppen) hergestellt gemäß Bückmann et al., Makromol. Chem. 182, 1379-1384 (1981), wurden in 1 ml destilliertem Wasser mit 5 µMol $N^6$-(2-Aminoethyl)-FAD bei pH 7.2 gelöst, eingestellt mit 0.1 N NaOH. Bei Zimmertemperatur wurde während einer Stunde bei pH 7.2 - 7.3 gerührt (0.1 N HCl oder 0.1 N NaOH). Das Reaktionsgemisch wurde gelfiltriert bei 4 °C über eine Sephadex G 50-Säule (0.5 x 60 cm), ins Gleichgewicht gegen destilliertes Wasser. Die Fraktion mit Polyethylenglykol $N^6$-(2-Aminoethyl)-FAD wurde eingeengt auf 1.7 ml mit 2 µMol an Polyethylenglykol($M_r$ = 20,000) gebundenem $N^6$-(2-Aminoethyl)-FAD [Konzentration 1.18 mM] mit Kopplungsausbeute 40 %.

D. Beispiel 2: Herstellung von CH-Sepharose 4B-$N^6$-(2-Aminoethyl)-FAD:

0.25 g CH-Sepharose 4B (Pharmacia, mit 10-14 µMol Carboxyhexylgruppen) gequollen in 3 ml destilliertem Wasser, wurden über einer Glasfritte mit 25 ml destilliertem Wasser gewaschen. Das halbtrokkene CH-Sepharose 4B wurde suspendiert in 0.5 ml destilliertem Wasser und 100 mg (520 µMol) 1-(3-Dimethylaminopropyl)-3-Ethyl-Carbodiimid-HCl zugegeben. Die Suspension wurde 10 min inkubiert bei Zimmertemperatur (pH-Einstellung auf 4.8-5.0 mit 0.1 N NaOH oder 0.1 N HCl). Das aktivierte CH-Sepharose 4B wurde innerhalb einer Minute zweimal mit 10 ml destilliertem Wasser gewaschen und addiert an 0.5 ml wäßriger Lösung mit 2.1 µMol $N^6$-(2-Aminoethyl)-FAD. Die Suspension wurde 16 Stunden bei Zimmertemperatur inkubiert nach pH-Einstellung auf 4.6 (0.1 N NaOH oder 0.1 N HCl).
Nach Waschen mit 30 ml 20 % LiCl und 20 ml destilliertem Wasser und Sedimentation in destilliertem Wasser wurden 1.2 ml CH-Sepharose 4B-$N^6$-(2-Aminoethyl)-FAD mit 1.75 µMol gebundenem $N^6$-(2-Aminoethyl)-FAD [Konzentration 1.45 mM] erhalten mit Kopplungsausbeute 83 %.

Literatur:


1. Mosbach, K., Advances in Enzymology 46, 205-278 (1978)
2. Wichmann, R., Wandrey, C., Bückmann, A. F. und Kula, M.-R., Biotechn. Bioeng. 23, 2789-2802 (1981)
3. Lindberg, M., Larsson, P.-O. und Mosbach, K., Eur. J. Biochem. 40, 187-193 (1973)
4. Lowe, C. R. und Mosbach, K., Eur. J. Biochem. 49, 511-520 (1974)
5. Muramatsu, M., Urabe. I., Yamada, Y. und Okada, H., Eur. J. Biochem 80, 111-117 (1977)
6. Zappelli, P., Rossodivita, A. und Re.L., Eur. J. Biochem. 54, 475-482 (1975)
7. Zappelli, P., Pappa, R., Rossodivita, A. und Re.L., Eur. J. Biochem. 72, 309-315 (1977)
8. Schmidt, H. L. und Grenner, G., Eur. J. Biochem. 67, 295-302 (1976)
9. Weibel, M. K., Fuller, C. W., Stadel, J. M., Bückmann, A. F. Doyle, T. und Bright, H. J., Enzyme Engineering 2, 203-208 (1974)
10. Yoshikawa, M., Goto, M., Ikura, K., Sasaki, R. und Chiba, H. Agric. Biol. Chem., 46, 207-213 (1982)
11. LeGoffic F., Sicsic, S. und Vincent, C., Eur. J. Biochem. 108, 143-148 (1980)
12. Fuller, C. W., Rubin, J.R. und Bright, H. J., Eur. J. Biochem. 103, 421-430 (1980)
13. Bückmann, A. F., Deutsches Patent 28.41.414 (1979)
14. Bückmann, A. F., Kula, M.-R., Wichmann, R. und Wandrey, C., J. Appl. Biochem. 3, 301-315 (1981)
15. Yamazaki, Y. und Maeda, H., Agric, Biol. Chem. 45, 2631-2632 (1981)
16. Zapelli, P., Pappa, R., Rossodivita, A. und Re L., Eur. J. Biochem. 89, 491-499 (1978)
17. Narasimhan, K. und Wingard, L. B., Appl. Biochem, Biotechn. 11, 221-232 (1985)


## Patentansprüche

**1.** Verfahren zur Herstellung von $N^6$-substituiertem NAD oder NADP, bei dem man
(a) NAD oder NADP mit Ethylenimin in N(1)-Stellung zu N(1)-(2-Aminoethyl)-NAD oder N(1)-(2-Aminoethyl)-NADP alkyliert,
(b) das Alkylierungsprodukt einer Dimroth-Umlagerung zu $N^6$-(2-Aminoethyl)-NAD oder $N^6$-(2-Aminoethyl)-NADP unterwirft und
(c) gegebenenfalls das gemäß (a) oder (b) erhaltene Produkt in an sich bekannter Weise kovalent an ein Makromolekül bindet,
dadurch ***gekennzeichnet,*** daß man
(d) in wässerigem Milieu umlagert,
(e) dabei einen pH-Wert von 4 bis 9 und insbesondere von 5 bis. 8 einhält und
(f) eine Temperatur von 25 bis 75 °C und insbesondere von 40 bis 60 °C einhält,
(g) der Dimroth-Umlagerung keine Reduktion vorausgehen und entsprechend keine Oxidation folgen läßt und
(h) das bei der Stufe (b) als Nebenprodukt gebildete 1,$N^6$-Aethanadenin-NAD bzw. 1,$N^6$-Aethanadenin-NADP abtrennt und isoliert.

**2.** Verfahren zur Herstellung von $N^6$-substituiertem FAD, bei dem man
(a) FAD in N(1)-Stellung alkyliert,
(b) das Alkylierungsprodukt ohne vorhergehende Reduktion und anschließende Reoxidation einer Dimroth-Umlagerung zum $N^6$-De-rivat unterwirft,
(c) gegebenenfalls das gemäß (a) oder (b) erhaltene Produkt in an sich bekannter Weise kovalent an ein Makromolekül bindet,
dadurch ***gekennzeichnet,*** daß man
(d) die Alkylierung mit Ethylenimin durchführt und das so erhaltene N(1)-(2-Aminoethyl)-FAD in wässrigem Milieu zum $N^6$-(2-Aminoethyl)-FAD umlagert,

(e) dabei einen pH-Wert von 4 bis 9 und insbesondere von 5 bis 8 einhält und
(f) eine Temperatur von 25 bis 75 °C und insbesondere von 40 bis 60 °C einhält, und
(g) das bei der Stufe (b) als Nebenprodukt gebildete 1,$N^6$-Aethanadenin-FAD abtrennt und isoliert.

**3.** Verfahren nach Anspruch 2, dadurch ***gekennzeichnet,*** daß ein pH-Wert von etwa 6.5, und eine Temperatur von etwa 40 °C eingehalten werden.

**Claims**

**1.** Process for the preparation of $N^6$-substituted NAD or NADP, in which
(a) NAD or NADP is alkylated with ethylene imine in the N(1)-position to N(1)-(2-aminoethyl)-NAD or N(1)-(2-aminoethyl)-NADP,
(b) the alkylation product is subjected to Dimroth rearrangement to form $N^6$-(2-aminoethyl)-NAD or $N^6$-(2-aminoethyl)-NADP and
(c) the product obtained according to (a) or (b) is optionally covalently bonded in a known manner to a macromolecule, characterised in that
(d) rearrangement is carried out in an aqueous medium,
(e) a pH of 4 to 9 and in particular 5 to 8 is maintained and
(f) a temperature of 25 to 75 °C and, in particular, of 40 to 60 °C is maintained,
(g) the Dimroth rearrangement is not preceded by a reduction and consequently not followed by oxidation and
(h) the 1,$N^6$-ethane adenine-NAD or 1,$N^6$-ethane adenine NADP formed as a by-product during stage (b) is separated and isolated.

**2.** Process for the preparation of $N^6$-substituted-FAD, in which
(a) FAD is alkylated in the N(1)-position,
(b) the alkylation product is subjected without prior reduction and subsequent reoxidation to a Dimroth rearrangement to form the $N^6$ derivative,
(c) the product obtained according to (a) or (b) is optionally covalently bonded in a known manner to a macromolecule, characterised in that
(d) alkylation is carried out with ethylene imine and the resultant N(1)-(2-aminoethyl)-FAD is rearranged in an aqueous medium to form $N^6$-(2-aminoethyl)-FAD,
(e) a pH of 4 to 9 and, in particular, of 5 to 8 is maintained and
(f) a temperature of 25 to 75 °C and, in particular, of 40 to 60 °C is maintained and
(g) the 1,$N^6$-ethane adenine-FAD formed as a by-product in stage (b) is separated and isolated.

**3.** Process according to claim 2, characterised in that a pH of about 6.5 and a temperature of about 40 °C are maintained.

**Revendications**

**1.** Procédé de préparation de NAD ou de NADP substitué en $N^6$, dans lequel
(a) on alkyle le NAD ou le NADP avec de l'éthylénimine en position N(1) pour donner le N(1)-(2-aminoéthyl)-NAD ou le N(1)-(2-aminoéthyl)-NADP,
(b) on soumet le produit de l'alkylation à une transposition de Dimroth pour donner le $N^6$-(2-aminoéthyl)-NAD ou le $N^6$-(2-aminoéthyl)-NADP, et
(c) le cas échéant, on lie par covalence d'une manière connue en soi le produit obtenu conformément à (a) ou (b) à une macromolécule,
caractérisé en ce que
(d) on effectue la transposition en milieu aqueux,
(e) on maintient pendant celle-ci un pH de 4 à 9, et en particulier de 5 à 8, et
(f) on maintient une température de 25 à 75 °C, et en particulier de 40 à 60 °C,
(g) on ne fait précéder la transposition de Dimroth d'aucune réduction et par conséquent on ne la fait suivre d'aucune oxydation, et
(h) on sépare le 1,$N^6$-éthanadénine-NAD ou le 1,$N^6$-éthanadénine-NADP formé comme sous-produit lors de l'étape (b) et on l'isole.

**2.** Procédé de préparation de FAD $N^6$-substitué, dans lequel

(a) on alkyle le FAD en position N(1),

(b) on soumet le produit de l'alkylation, sans réduction préalable ni réoxydation ultérieure, à une transposition de Dimroth pour donner le dérivé en $N^6$,

(c) le cas échéant, on lie par covalence d'une manière connue en soi, le produit obtenu conformément à (a) ou (b) sur une macromolécule,

caractérisé en ce que

(d) on effectue l'alkylation avec de l'éthylénimine et on transpose le N(1)-(2-aminoéthyl)-FAD ainsi obtenu, en milieu aqueux, pour donner le $N^6$ -(2-aminoéthyl)FAD,

(e) on maintient pendant cette opération un pH de 4 à 9, et en particulier de 5 à 8, et

(f) on maintient une température de 25 à 75 °C, et en particulier de 40 à 60 °C,

(g) on sépare le 1,$N^6$-éthanadénine-FAD formé comme sous-produit lors de l'étape (b) et on l'isole.

**3.** Procédé selon la revendication 2, caractérisé en ce qu'on maintient un pH d'environ 6,5 et une température d'environ 40 °C.